Europäisches Patentamt

European Patent Office

Office européen des brevets

(11) Veröffentlichungsnummer: **0 465 783 A1**

## (12) EUROPÄISCHE PATENTANMELDUNG

(21) Anmeldenummer: **91106895.5**

(22) Anmeldetag: **27.04.91**

(51) Int. Cl.5: **A61M 15/02, A61N 1/44, H01T 23/00**

(30) Priorität: **13.07.90 DE 4022393**

(43) Veröffentlichungstag der Anmeldung:
**15.01.92 Patentblatt 92/03**

(84) Benannte Vertragsstaaten:
**AT BE CH DE DK ES FR GB GR IT LI LU NL SE**

(71) Anmelder: **Marquardt, Klaus**
**Am Sandrain 10**

**W-7522 Philippsburg 2(DE)**

(72) Erfinder: **Marquardt, Klaus**
**Am Sandrain 10**
**W-7522 Philippsburg 2(DE)**

(74) Vertreter: **Ratzel, Gerhard, Dr.**
**Seckenheimer Strasse 36a**
**W-6800 Mannheim 1(DE)**

(54) **Ionisator zur Ionisierung von Sauerstoff bei Sauerstoff-Therapie.**

(57) Die Erfindung betrifft einen Ionisator zur Ionisierung von Sauerstoff bei Sauerstoff-Therapien, bei der dieser ein die Ionisationseinheit aufnehmendes Gehäuse aufweist, auf das mittels eines leitenden Metallringes ein mittels eines hochohmigen Widerstandes mit Masse verbundenes Berührungsschutzgitter aufgesetzt ist, wobei als Verbindung zwischen Atemmaske und Ionisator ein Glaskolben auf das Gehäuse aufgebracht bzw. aufgeschraubt ist. Das Gehäuse ist hierbei an einem endseitigen Stutzen von einem leitenden Metallzylinder umgeben, der mit einem hochohmigen Widerstand in Verbindung steht, welcher wiederum eine Verbindung mit dem Berührungsschutzgitter aufweist. Die Ionisationseinheit ist innerhalb des Gehäuses derart angeordnet, daß sich die Ionisationsnadeln unmittelbar unter dem Berührungsschutzgitter befinden.

Fig.1

EP 0 465 783 A1

Rank Xerox (UK) Business Services

Die Erfindung betrifft einen Ionisator zur Ionisierung von Sauerstoff bei der Anwendung aller Sauerstoff-Therapien.

Die ionisierte Sauerstoff-Inhalations-Therapie (IO2ITh) ist eine kurzzeitige Inhalation mit einer definierten Mischung von medizinischem Sauerstoff und wahlweise negativen oder positiven Sauerstoffionen zu therapeutischen oder präventiven Zwekken. Es handelt sich bei der IO2Ith um eine partielle und polare Ionisation des medizinischen Sauerstoffes mit einem geeigneten Sauerstoffionisationsgerät für Inhalationstherapien.

Die auf dem Markt befindlichen Ionisatoren für die Sauerstoffionisierung produzieren zwar fast alle ausnahmslos mehrere Millionen Ionen pro cm$^3$/sec. Die Inhalationsmaske wird jedoch lediglich durch eine ungenügende Menge dieser Ionen erreicht, da im Zufuhrschlauch zwischen Ionisator und Atemmaske über 90% der Ionen entladen werden. Bei stärkerer Druckspannungs-Zuführung werden zwar mehr Ionen frei, jedoch entsteht hierbei als Nebenprodukt Ozon, welches als Atemgift einzuordnen ist.

Ionisatoren, welche direkt vor der Maske ionisieren, um dieses Problem zu lösen, verwenden als Berührungsschutz Kunststoff. Kunststoff, gleich welcher Art, vernichten die Ionisierung.
Daher sind solche Lösungen als unbefriedigend einzustufen.

Der vorliegenden Erfindung liegt daher die Aufgabe zugrunde, einen Ionisator zu schaffen, bei der die Ionisation direkt vor der Atemmaske stattfindet, wobei im Ionisationsbereich bis zur Maske kein Kunststoff verwendet wird, damit genügend Ionen vor der Atemmaske vorhanden sind, ohne Ozon zu enthalten.

Diese Aufgabe wird erfindungsgemäß durch einen Ionisator gemäß des vorgeschlagenen Anspruchs 1 gelöst.

Besondere Ausführungsmerkmale der Erfindung sind dabei in den Unteransprüchen gekennzeichnet.

Bei dem erfindungsgemäßen Ionisator kann am Ende die Atemmaske direkt aufgesteckt werden. Der Berührungsschutz ist aus Edelstahl und durch einen hochohmigen Widerstand mit Masse verbunden. Die angelegte Hochspannung liegt unter dem Bereich, indem Ozon entsteht. Für die Maskenhalterung wurde Glas als Material herangezogen.

Hierbei ist vorgesehen, daß der Ionisator ein die Ionisationseinheit aufnehmendes Gehäuse aufweist, auf das mittels eines leitenden Metallringes ein mittels eines hochohmigen Widerstandes mit Masse verbundenes Berührungsschutzgitters aufgesetzt ist, wobei als Verbindung zwischen Atemmaske und Ionisator ein Glaskolben auf das Gehäuse aufbringbar bzw. aufschraubbar ist.

Das Gehäuse weist einen endseitigen Stutzen auf, der von einem leitenden Metallzylinder umgeben ist. Innerhalb des Gehäuses ist ein Hohlraum vorgesehen, in dem die Ionisationseinheit angeordnet ist.

Die Ionisationsnadeln sind unmittelbar unterhalb des Berührungsschutzgitters angeordnet, wobei sich die Ionisationsnadeln auf oder in einer Aufnahmehalterung befinden, der sich eine Aufnahmebuchse für ein Hochspannungskabel anschließt.

Im Gehäuseboden sind Sauerstoffdurchtrittsbohrungen angeordnet, sowie die Aufnahmebuchse für das Hochspannungskabel durchgeführt und in diesen oder an diesen befestigt.

Zwischen Metallring und Berührungsschutzgitter liegt eine feste Verbindung vor, die vorzugsweise mittels einer Lötstelle geschaffen wird.

Besonders bevorzugtes Material für die Ionisationsnadeln und für die Aufnahmebuchse ist V2A-Stahl.

Auch das Berührungsschutzgitter ist aus einem Edelstahlmaterial geschaffen.

Anhand der beigefügten Zeichnung, die ein besonders bevorzugtes Ausführungsbeispiel der Erfindung zeigt, wird diese nun näher erläutert.

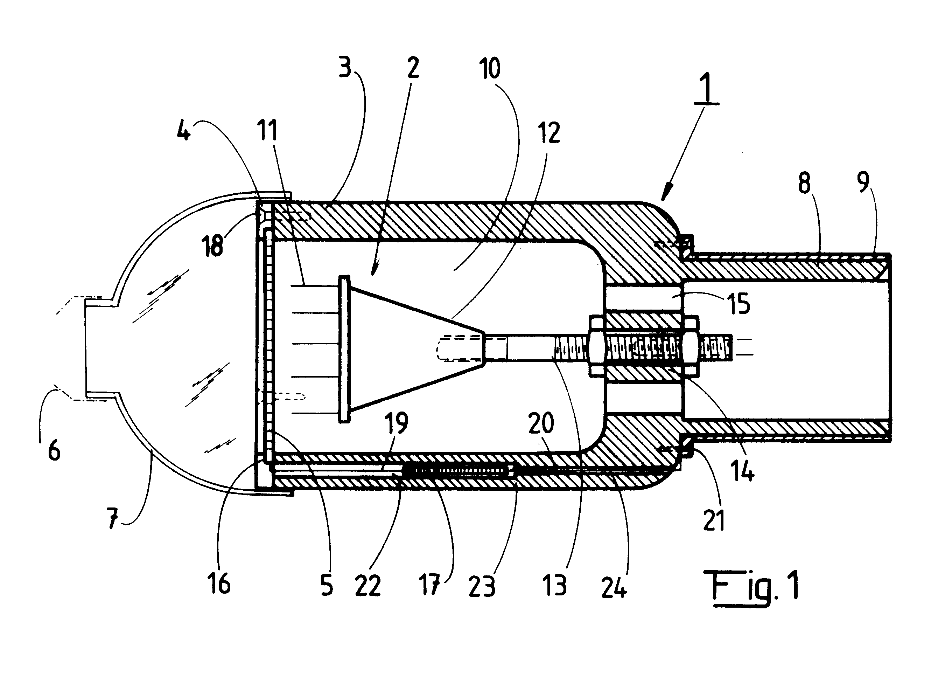

Die Figur 1 zeigt den erfindungsgemäßen Ionisator 1. Dieser besteht aus einem Gehäuse 3 mit einem Hohlraum 10. Das Gehäuse ist endseitig verjüngt und weist somit einen abstehenden Stutzen 8 auf, der von einem leitenden Metallzylinder 9 umgeben ist. Der Gehäuseboden 14 weist zum Durchströmen des Sauerstoffs Durchtrittsbohrungen 15 auf. Durch den Gehäuseboden 14 ist eine Aufnahmebuchse 13 für ein Hochspannungskabel geführt, dem sich eine Aufnahmehalterung 12 für die Ionisationsnadeln 11 anschließt. Diese sind wiederum unmittelbar unter einem Berührungsschutzgitter 5 angeordnet, welche mittels eines leitenden Metallringes 4 durch entsprechende Schrauben 18 auf dem Gehäuse 3 befestigt ist/sind.

Metallring 4 und Berührungsschutzgitter 5 weisen eine feste Verbindung auf, die vorzugsweise mittels einer Lötstelle 16 geschaffen ist.

Von hier aus führt ein entsprechendes Kabel 19 zu einem hochohmigen Widerstand 17, der wiederum eine Kabelverbindung 20 mit dem leitenden Metallzylinder 9 aufweist, welcher mittels Schrauben 21 am Gehäuse 3 befestigt ist.

Der hochohmige Widerstand 17 ist in diesem Ausführungsbeispiel innerhalb einer Tasche 22 der Gehäusewand 23 eingesetzt, wobei sich dieser Tasche 22 eine Durchgangsbohrung 24 zur Durchführung des Kabels 20 zur Verbindung mit dem Metallzylinder 9 anschließt.

Die strichpunktiert angedeutete Atemmaske 6 wird auf den Glaskolben 7 aufgesteckt. Der Glaskolben 7 selbst wird mit dem Gehäuse 3 mittels Verschraubung oder gleichartiger Verbindung zusammengebracht, wobei auf geeignete Dichtungsmittel nicht näher eingegangen wird.

Mit der vorliegenden Erfindung wird ein Ionisator geschaffen, bei dem durch die Verwendung der erfindungsgemäßen Einzelteile und deren Anordnung eine effiziente Ausbeute an ionisiertem Sauerstoff an der Atemmaske und somit zur Inhalation bereitsteht, wobei als Berührungsschutz kein Kunststoff verwendet wird, welcher die Ionisierung vernichtet und die Ozonbildung vermieden wird.

**Patentansprüche**

1. Ionisator zur Ionisierung von Sauerstoff bei Sauerstoff-Therapien,
dadurch gekennzeichnet,
daß dieser, ein die Ionisationseinheit (2) aufnehmendes Gehäuse (3) aufweist, auf das mittels eines leitenden Metallringes (4) ein, mittels eines hochohmigen Widerstandes (17) mit Masse verbundenes Berührungsschutzgitter (5) aufgesetzt ist, wobei als Verbindung zwischen Atemmaske (6) und Ionisator (1) ein Glaskolben (7) auf das Gehäuse aufgebracht bzw. aufgeschraubt ist.

2. Ionisator nach Anspruch 1,
dadurch gekennzeichnet,
daß das Gehäuse (3) einen endseitigen Stutzen (8) aufweist, der von einem leitenden Metallzylinder (9) umgeben ist.

3. Ionisator nach Anspruch 1 bis 2,
dadurch gekennzeichnet,
daß das Gehäuse (3) einen Hohlraum (10) aufweist, innerhalb dem die Ionisationseinheit (2) angeordnet ist.

4. Ionisator nach Anspruch 1 bis 3,
dadurch gekennzeichnet,
daß unmittelbar unterhalb des Berührungsschutzgitters (5) die Ionisationsnadeln (11) angeordnet sind.

5. Ionisator nach Anspruch 1 bis 4,
dadurch gekennzeichnet,
daß sich die Ionisationsnadeln (11) auf/in einer Aufnahmehalterung (12) befinden.

6. Ionisator nach Anspruch 1 bis 5,
dadurch gekennzeichnet,
daß sich der Aufnahmehalterung (12) eine Aufnahmebuchse (13) für ein Hochspannungskabel anschließt.

7. Ionisator nach Anspruch 1 bis 6,
dadurch gekennzeichnet,
daß im Gehäuseboden (14) Sauerstoffdurchtrittsbohrungen (15) angeordnet sind.

8. Ionisator nach Anspruch 1 bis 7,
dadurch gekennzeichnet,
daß die Aufnahmebuchse (13) durch den Gehäuseboden (14) geführt und in/an diesem befestigt ist.

9. Ionisator nach Anspruch 1 bis 8,
dadurch gekennzeichnet,
daß zwischen Metallring (4) und Berührungsschutzgitter (5) eine feste Verbindung vorliegt.

10. Ionisator nach Anspruch 9,
dadurch gekennzeichnet,
daß die feste Verbindung in Form einer Lötstelle (16) ausgebildet ist.

11. Ionisator nach Anspruch 1 und mindestens einem der vorhergehenden Ansprüche,
dadurch gekennzeichnet,
daß die Ionisationsnadeln (11) aus V2A-Stahl geschaffen sind.

12. Ionisator nach Anspruch 1 und mindestens einem der vorhergehenden Ansprüche,
dadurch gekennzeichnet,
daß die Aufnahmebuchse (13) aus V2A-Stahl geschaffen ist.

13. Ionisator nach Anspruch 1 und mindestens einem der vorhergehenden Ansprüche,
dadurch gekennzeichnet,
daß das Berührungsschutzgitter (5) aus Edelstahl geschaffen ist.

Fig. 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,X | DE-U-9 010 571 (MARQUARDT) <br> * das ganze Dokument * <br> – – – | 1-13. | A 61 M 15/02 <br> A 61 N 1/44 <br> H 01 T 23/00 |
| A | US-A-3 096 762 (WINCHELL) <br> * Spalte 2, Zeile 46 - Spalte 3, Zeile 40; Figur 1 * <br> – – – | 1. | |
| A | DE-U-8 529 413 (EMC-ERZEUGNISSE FUER MEDIZIN & CHIRURGIE.) <br> – – – – – | | |

|  | RECHERCHIERTE SACHGEBIETE (Int. Cl.5) |
|---|---|
|  | A 61 M <br> A 61 N <br> H 01 T |

Der vorliegende Recherchenbericht wurde für alle Patentansprüche erstellt

| Recherchenort | Abschlußdatum der Recherche | Prüfer |
|---|---|---|
| Den Haag | 12 September 91 | BIJN E.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument
---------------------------------------------------------
& : Mitglied der gleichen Patentfamilie, übereinstimmendes Dokument